(19) Europäisches Patentamt
European Patent Office
Office européen des brevets

(11) Veröffentlichungsnummer : **0 037 009**
**B1**

(12) # EUROPÄISCHE PATENTSCHRIFT

(45) Veröffentlichungstag der Patentschrift : **05.10.83**

(51) Int. Cl.³ : **A 61 K 7/08**, A 61 K 7/50

(21) Anmeldenummer : **81102001.5**

(22) Anmeldetag : **18.03.81**

(54) **Haarwasch- und Körperreinigungsmittel.**

(30) Priorität : **26.03.80 DE 3011549**

(43) Veröffentlichungstag der Anmeldung : **07.10.81 Patentblatt 81/40**

(45) Bekanntmachung des Hinweises auf die Patenterteilung : **05.10.83 Patentblatt 83/40**

(84) Benannte Vertragsstaaten : **AT BE CH DE FR GB IT LI LU NL SE**

(56) Entgegenhaltungen :
**FR A 2 087 001**
**FR A 2 252 840**
**US A 3 819 828**
**US A 4 137 191**

**H. JANISTYN : « Handbuch der Kosmetika und Riechstoffe », 2. Auflage, Band 3, 1973, Dr. Alfred Hüthig Verlag, Seiten 254-255 Heidelberg, DE. « Die Körperpflegemittel »**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Ansmann, Achim, Dr.**
**Fichtestrasse 19**
**D-4010 Hilden (DE)**
Erfinder : **Guirr, Ortburg geb. Düsterloh**
**Eichhornstrasse 68**
**D-4150 Krefeld 1 (DE)**
Erfinder : **Weichbrodt, Maria, Dr. geb. Nolte**
**Karl-Leverkus-Strasse 1**
**D-5074 Odenthal (DE)**

**EP 0 037 009 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Haarwasch- und Körperreinigungsmittel

Die Erfindung betrifft Haarwasch- und Körperreinigungsmittel auf Basis von Alkylethersulfaten mit verbesserter Haut- und Schleimhautverträglichkeit.

Für die Herstellung von Haarwasch-, Körperreinigungs- und Handwaschmitteln, die nicht auf Seifenbasis aufgebaut waren, hat man seit langem oberflächenaktive Alkylsulfatsalze als hauptsächliches Tensid eingesetzt. Derartige Alkylsulfatsalze konnten aber im Hinblick auf Hautfreundlichkeit und Schleimhautverträglichkeit nicht befriedigen. Eine gewisse Verbesserung konnte durch den Einsatz von speziellen Alkylethersulfaten, insbesondere in Kombination mit oberflächenaktiven Betainen erzielt werden. Dabei handelt es sich bei den Alkylethersulfaten um Produkte auf Basis von Fettalkoholen, die etwa 70 % Dodecylalkohol neben 30 % überwiegend $C_{14}$-Fettalkohol enthalten. So ist zum Beispiel aus FR-A-2.252.840 ein Shampoo bekannt, welches 6 g eines Myristylethersulfats und 9 g eines Laurylethersulfats enthält. Aus US-A-3.819.828 ist die Verwendung eines Fettalkoholsulfatgemisches mit 50 % $C_{12}$ und 50 % $C_{14}$-Alkylketten in wasserfreien, oxidativen Haarbehandlungsmitteln bekannt. Die Verwendung von Gemischen aus Betaintensiden und Alkylethersulfaten in flüssigen Reinigungsmitteln ist z. B. aus FR-A-2.087.001 bekannt. In US-A-4.137.191 werden wasserfreie Lösungen von Betaintensiden und Fettalkoholsulfaten oder Ethersulfaten in wasserlöslichen polaren Solventien vorgeschlagen. Aus « The Journal of the American Oil Chemist' s Society, Band 45, Nr. 11 (November 1968), Seite 738 sind Alkoholethersulfate unterschiedlicher C-Kettenverteilung als Shampootenside bekannt. Diese Kombinationen ließen hinsichtlich ihrer Schleimhautreizung noch Wünsche offen, so daß das Bedürfnis nach verbesserten Haarwasch- und Körperreinigungsmitteln, die bei hoher Reinigungswirkung keine Schleimhautreizung bewirken, weiterhin bestand.

Es wurde nun gefunden, daß Haarwasch- und Körperreinigungsmittel auf Basis von Alkylethersulften mit einem Gehalt an einem Alkylethersulfatgemisch, bestehend aus 50 Gewichtsprozent eines $C_{12}$-Alkylethersulfats und 50 Gewichtsprozent eines $C_{14}$-Alkylethersulfats mit 1 bis 5, vorzugsweise 2 bis 4 Ethylenoxidgruppen als Etherkomponente, die gestellten Forderungen weitgehend erfüllen.

Die erfindungsgemäß einzusetzenden Alkylethersulfatgemische mit je 50 Gewichtsprozent $C_{12}$- und $C_{14}$-Alkylethersulfat sind in ihrer Haut- und Schleimhautverträglichkeit nicht nur wesentlich besser als die bisher eingesetzten Alkylethersulfate mit 70 % $C_{12}$- neben 30 % $C_{14}$-Anteil, sondern sie sind überraschenderweise auch wesentlich besser als die Gemische der bisher eingesetzten Alkylethersulfate mit oberflächenaktiven Betainen, ohne die gute Waschkraft der Haarwasch- und Körperreinigungsmittel zu mindern.

Durch geringfügige Anteile anderer C-Kettenlänge wird die überraschend gute Schleimhaut- und Hautverträglichkeit der erfindungsgemäß zu verwendenden Produkte nur unwesentlich verändert.

Durch den Zusatz von Betainen läßt sich die ausgezeichnete Haut- und Schleimhautverträglichkeit der erfindungsgemäß einzusetzenden Alkylethersulfatgemische noch steigern. Als amphotere oberflächenaktive Betaine können dabei Verbindungen der Formel

$$R_1 - \overset{\overset{R_2}{|}}{\underset{\underset{R_3}{|}}{N^{(+)}}} - CH_2COO^{(-)}$$

in der $R_1$ einen gegebenenfalls durch Heteroatome oder Heteroatomgruppen unterbrochenen Alkylrest mit 8-20 und $R_2$ und $R_3$ gleichartige oder verschiedene Alkylreste mit 1-3 Kohlenstoffatomen bedeuten, Verwendung finden. Als besonders geeignete Betaine haben sich hierbei das $C_8$-$C_{18}$-Alkyl-dimethylcarboxymethylbetain und das $C_{12}$-$C_{18}$-Alkyl-amidopropyldimethylcarboxymethylbetain erwiesen.

Die erfindungsgemäßen Haarwasch- und Körperreinigungsmittel enthalten das Alkylethersulfatgemisch, bestehend aus 50 Gewichtsprozent eines $C_{12}$-Alkylethersulfats und 50 Gewichtsprozent eines $C_{14}$-Alkylethersulfats mit 1 bis 5, vorzugsweise 2 bis 4 Ethylenoxidgruppen als Etherkomponente, in einer Menge von 1-40 Gewichtsprozent, vorzugsweise 5-25 Gewichtsprozent, bezogen auf das gesamte Mittel. Die zur weiteren Verbesserung von Haut-, Schleimhautverträglichkeit und Waschwirksamkeit zusätzlich zu verwendenden Betaine, insbesondere $C_8$-$C_{18}$-Alkyl-dimethylcarboxymethylbetain und $C_{12}$-$C_{18}$-Alkyl-amidopropyldimethylcarboxymethylbetain, sind in einer Menge von 0,1-20 Gewichtsprozent, vorzugsweise 0,5-5 Gewichtsprozent, bezogen auf das gesamte Mittel, enthalten.

Das erfindungsgemäß einzusetzende Alkylethersulfatgemisch, in Kombination mit den Betainen, kann selbstverständlich auch in höherer Konzentration, ja sogar als wasserfreies Gemisch auf den Markt gebracht werden, wobei es dann von dem Weiterverarbeiter mit einem entsprechenden wäßrigen oder wäßrig-alkoholischen Medium auf eine Gebrauchskonzentration verdünnt werden muß.

Da von einem Körperreinigungsmittel, insbesondere einem Haarwaschmittel die Bildung einer reichlichen Schaummenge erwartet wird, ist es empfehlenswert, den Kompositionen einen Schaumstabilisator hinzuzufügen. Als Schaumstabilisatoren können Alkylolamide der allgemeinen Formel

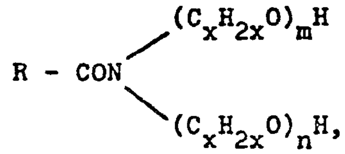

in der R einen Alkylrest mit 8 bis 18 Kohlenstoffatomen, x die Zahlen 2 oder 3, m eine Zahl von 1 bis 5 und n eine Zahl von 0 bis 5 bedeuten, eingesetzt werden. Bevorzugte Schaumstabilisatoren stellen dabei das Monoethanolamid, Monoisopropanolamid, Diethanolamid und Diisopropanolamid der entsprechenden Fettsäuren, insbesondere der Kokosnußfettsäuren dar.

Als Schaumstabilisatoren können ferner tertiäre Aminoxide, die eine hydrophobe Gruppe aufweisen, dienen wie zum Beispiel Lauryldimethylaminoxid, Lauryl-bis-(2-hydroxy-ethyl)-aminoxid, Lauryl-trioxyethylen-dimethylaminoxid und andere mehr.

Der Gehalt der erfindungsgemäßen Haarwasch- und Körperreinigungsmittel an Schaumstabilisatoren beträgt 0 bis 20 Gewichtsprozent, vorzugsweise 1 bis 5 Gewichtsprozent, bezogen auf das gesamte Mittel.

Darüber hinaus können die erfindungsgemäßen Haarwasch- und Körperreinigungsmittel weitere Hilfsmittel wie Antischuppenwirkstoffe, Haarpflegemittel, Trübungsmittel, Rückfettungsmittel, Lösungsvermittler, Parfüm, Farbstoffe, germicide Mittel und Konservierungsmittel in den für derartige Produkte üblichen Mengen enthalten.

Die nachfolgenden Beispiele sollen den Gegenstand der Erfindung näher erläutern, ohne ihn darauf zu beschränken.

## Beispiele

Zum Nachweis der außergewöhnlich guten Schleimhautverträglichkeit der erfindungsgemäß einzusetzenden Substanzen wurde der von J. H. Draize in « Appraisal of the Safety of Chemicals in Foods, Drugs and Cosmetics, Ass. of Food and Drug officials of the US » (1959), Seite 49-52 beschriebene Test am Kaninchenauge durchgeführt. Dabei wurden 2 Rezepturen nach dem Stand der Technik und 2 Rezepturen mit den erfindungsgemäß zu verwendenden Produkten in unverdünnter Form eingesetzt. Die Kontaktzeit betrug 10 Sekunden.

| | |
|---|---|
| Rezeptur 1 gemäß Stand der Technik | |
| $C_{12-14}$-Alkyl-2EO-sulfat (70 $C_{12}$/30 $C_{14}$) | 20 Gewichtsteile |
| Wasser | 80 Gewichtsteile |
| Rezeptur 2 | |
| $C_{12-14}$-Alkyl-3-EO-sulfat (50 $C_{12}$/50 $C_{14}$) | 20 Gewichtsteile |
| Wasser | 80 Gewichtsteile |
| Rezeptur 3 gemäß Stand der Technik | |
| $C_{12-14}$-Alkyl-2EO-sulfat (70 $C_{12}$/30 $C_{14}$) | 18 Gewichtsteile |
| $C_8$-$C_{18}$-Alkyl-dimethylcarboxymethylbetain | 2 Gewichtsteile |
| Wasser | 80 Gewichtsteile |
| Rezeptur 4 erfindungsgemäß | |
| $C_{12-14}$-Alkyl-3-EO-sulfat (50 $C_{12}$/50 $C_{14}$) | 18 Gewichtsteile |
| $C_8$-$C_{18}$-Alkyl-dimethylcarboxymethylbetain | 2 Gewichtsteile |
| Wasser | 80 Gewichtsteile |

Die nachstehend aufgeführte Tabelle 1 mit den Ergebnissen des Tests über die Schleimhautverträglichkeit am Kaninchenauge zeigt deutlich die überraschend hohe Verbesserung der Schleimhautverträglichkeit durch die erfindungsgemäß zu verwendenden Produkte gegenüber den Verbindungen des Standes der Technik. Bereits der alleini-ege Einsatz der erfindungsgemäß einzusetzenden Produkte führt zu wesentlich besseren Werten als die aus dem Stand der Technik bekannte Kombination mit Betainen.

(Siehe Tabelle 1 Seite 4 f.)

Tabelle 1

| | Durchschnittliche conjunctivale Reaktion in % der maximal möglichen Reaktion nach | | | | | |
|---|---|---|---|---|---|---|
| | 2 h | 6 h | 24 h | 48 h | 72 h | 96 h |
| Rezeptur 1 | 55 | 60 | 35 | 15 | 2,5 | 0 |
| Rezeptur 2 | 30 | 25 | 2,5 | 0 | 0 | 0 |
| Rezeptur 3 | 45 | 45 | 17,5 | 17,5 | 5 | 0 |
| Rezeptur 4 | 25 | 20 | 2,5 | 0 | 0 | 0 |

Ferner wurde die Hautverträglichkeit der vorgenannten 4 Rezepturen nach dem Hautirritationstest bestimmt, wie er von Peter J. Frosch und Albert M. Kligman als « The soap chamber test » im Journal of the American Academy of Dermatology, Volume I, Number I, July 1979, Seite 35-41 beschrieben ist. Die einzelnen Rezepturen wurden dabei in einer Konzentration mit 0,5 % Aktivsubstanz eingesetzt. Die bei diesem Test erhaltenen Werte sind der nachfolgenden Tabelle 2 zu entnehmen.

Tabelle 2

| | Hautirritationsindex | | |
|---|---|---|---|
| | Erythem | Schuppung | gesamt |
| Rezeptur 1 | 0,4 | 0,7 | 1,1 |
| Rezeptur 2 | 0 | 0,1 | 0,1 |
| Rezeptur 3 | 0,4 | 0,6 | 1,0 |
| Rezeptur 4 | 0 | 0 | 0 |

Wie der Tabelle 2 zu entnehmen ist, zeigen sich die erfindungsgemäß einzusetzenden Produkte, denen des Standes der Technik weit überlegen, selbst dann, wenn die des Standes der Technik in Kombination mit Betainen zur Verbesserung der Hautverträglichkeit eingesetzt werden.

Nachfolgend werden noch einige Rezepturen für erfindungsgemäße Haarwasch- und Körperreinigungsmittel aufgeführt.

Haarwaschmittel

| | |
|---|---|
| $C_{12}/C_{14}$-Alkyl-3 EO-sulfat (50 $C_{12}$/50 $C_{14}$) | 12,0 Gewichtsteile |
| $C_{12}$-$C_{18}$-Alkyl-amidopropyl-dimethylcarboxymethylbetain | 5,0 Gewichtsteile |
| Kokosfettsäurediethanolamid | 1,5 Gewichtsteile |
| Wasser, Natriumchlorid, Parfüm | ad 100,0 Gewichtsteile |

Haarwaschmittel

| | |
|---|---|
| $C_{12}/C_{14}$-Alkyl-3-EO-sulfat (50 $C_{12}$/50 $C_{14}$) | 20,0 Gewichtsteile |
| $C_8$-$C_{18}$-Alkyl-dimethylcarboxymethylbetain | 2,0 Gewichtsteile |
| Kokosfettsäurediisopropanolamid | 2,0 Gewichtsteile |
| Wasser, Natriumchlorid, Parfüm | ad 100,0 Gewichtsteile |

Schaumbad

| | |
|---|---|
| $C_{12}/C_{14}$-Alkyl-2EO-sulfat (50 $C_{12}$/50 $C_{14}$) | 30,0 Gewichtsteile |
| $C_8$-$C_{18}$-Alkyl-dimethylcarboxymethylbetain | 5,0 Gewichtsteile |
| Fichtennadelöl | 5,0 Gewichtsteile |
| Ester von Glycerin-Ethylenoxidaddukten mit langkettigen Fettsäuren als Rückfettungsmittel | 10,0 Gewichtsteile |
| Wasser, Parfüm, Farbstoff | ad 100,0 Gewichtsteile |

Duschbad

| | |
|---|---|
| $C_{12}/C_{14}$-Alkyl-2EO-sulfat (50 $C_{12}$/50 $C_{14}$) | 15,0 Gewichtsteile |
| $C_8$-$C_{18}$-Alkyl-dimethylcarboxymethylbetain | 2,0 Gewichtsteile |
| Kokosfettsäuremonoethanolamid | 3,0 Gewichtsteile |
| Ester von Glycerin-Ethylenoxidaddukten mit langkettigen | |

| | |
|---|---|
| Fettsäuren als Rückfettungsmittel | 5,0 Gewichtsteile |
| Wasser, Natriumchlorid, Parfüm, Farbstoff | ad 100,0 Gewichtsteile |

## Ansprüche

1. Haarwasch- und Körperreinigungsmittel auf Basis von Alkylethersulfaten und amphoteren oberflächenaktiven Betainen, dadurch gekennzeichnet, daß 1-40 Gewichtsprozent eines Alkylethersulfatgemisches, bestehend aus 50 Gewichtsprozent eines $C_{12}$-Alkylethersulfats und 50 Gewichtsprozent eines $C_{14}$-Alkylethersulfats mit 1 bis 5 Ethylenoxidgruppen als Etherkomponente und 0,1 bis 20 Gewichtsprozent eines amphoteren, oberflächenaktiven Betains enthalten ist.

2. Haarwasch- und Körperreinigungsmittel nach Anspruch 1, dadurch gekennzeichnet, daß 2-25 Gewichtsprozent des Alkylethersulfatgemisches und 0,5-5 Gewichtsprozent des amphoteren oberflächenaktiven Betains enthalten ist.

3. Haarwasch- und Körperreinigungsmittel nach Anspruch 1 und 2, dadurch gekennzeichnet, daß als Betain ein $C_8$-$C_{18}$-Alkyl-dimethylcarboxymethylbetain und/oder $C_{12}$-$C_{18}$-Alkylamidopropyl-dimethylcarboxymethylbetain enthalten ist.

## Claims

1. A hair and body shampoo based on alkyl ether sulfates and amphoteric surface-active betaines, characterized in that it contains from 1 to 40 % by weight of an alkyl ether sulfate mixture, consisting of 50 % by weight of a $C_{12}$-alkyl ether sulfate and 50 % by weight of a $C_{14}$-alkyl ether sulfate containing from 1 to 5 ethylene oxide groups, as the ether component and from 0.1 to 20 % by weight of an amphoteric, surface-active betaine.

2. A hair and body shampoo as claimed in Claim 1, characterized in that it contains from 2 to 25 % by weight of the alkyl ether sulfate mixture and from 0.5 to 5 % by weight of the amphoteric surface-active betaine.

3. A hair and body shampoo as claimed in Claims 1 and 2, characterized in that it contains a $C_8$-$C_{18}$-alkyl dimethyl carboxymethyl betaine and/or a $C_{12}$-$C_{18}$-alkylamidopropyl dimethylcarboxymethyl betaine as the betaine.

## Revendications

1. Produit pour le lavage des cheveux et le nettoyage du corps, à base de sulfates d'éthers alkyliques et de bétaïnes tensio-actives amphotères, caractérisé en ce qu'il contient de 1 à 40 % en poids d'un mélange de sulfate d'éthers alkyliques consistant en 50 % en poids d'un sulfate d'éther alkylique en $C_{12}$ et 50 % en poids d'un sulfate d'éther alkylique en $C_{14}$ contenant 1 à 5 groupes oxyde d'éthylène en tant que composant éther et 0,1 à 20 % en poids d'une bétaïne tensio-active amphotère.

2. Produit pour le lavage des cheveux et le nettoyage du corps selon la revendication 1, caractérisé en ce qu'il contient de 2 à 25 % en poids du mélange de sulfates d'éthers alkyliques et de 0,5 à 5 % en poids de la bétaïne tensio-active amphotère.

3. Produit pour le lavage des cheveux et le nettoyage du corps selon les revendications 1 à 2, caractérisé en ce qu'il contient en tant que bétaïne une (alkyle en $C_8$-$C_{18}$)-diméthylcarboxyméthylbétaïne et/ou une (alkyle en $C_{12}$-$C_{18}$)-amidopropyldiméthylcarboxyméthylbétaïne.